# EUROPEAN PATENT APPLICATION

(11) **EP 2 351 563 A1**
(43) Date of publication of application: **03.08.2011**
(21) Application number: 09823532.8
(22) Date of filing: 26.10.2009
(51) Int. Cl.: A61K 31/40, A61K 31/155, A61K 31/4985, A61K 31/64, A61K 38/00, A61K 38/28, A61K 45/00, A61P 1/04, A61P 1/16, A61P 3/04, A61P 3/10, A61P 11/00, A61P 11/06, A61P 25/00, A61P 25/16, A61P 29/00, A61P 35/00, A61P 37/02, A61P 43/00

(54) **AGENT FOR TREATMENT OF DIABETES**

(30) Priority: 31.10.2008 JP 2008280815
(71) Applicant: Dainippon Sumitomo Pharma Co., Ltd., Osaka 541-8524 (JP)
(72) Inventor: NAGAMINE, Jun, Osaka-shi Osaka 554-0022 (JP)
(74) Representative: Vossius & Partner
(86) International application number: PCT/JP2009/068312
(87) International publication number: WO 2010/050422

(57) **Abstract**

The present invention provides a hypoglycemic agent useful for treating diabetes or other similar diseases which has no adverse effects. Furthermore, the present invention provides a hypoglycemic agent comprising a combination of an antidiabetic drug and 2-methyl-2-[(4-{(1E)-3-[2-(4-methylbenzoyl)-1H-pyrrol-1-yl]prop-1-en-1-yl}benzyl)oxy]propanoic acid or a pharmaceutically acceptable salt thereof.

## Description

### TECHNICAL FIELD

The present invention relates to a hypoglycemic agent useful for treating diabetes or other similar diseases comprising a combination of a heteroaryl derivative having an insulin-sensitizing action and an antidiabetic drug.

### BACKGROUND ART

Diabetes is one of metabolic syndromes whose cardinal sign is a state of chronic hyperglycemia caused by the insufficient insulin action; and it is known that one of the causes thereof is a shortage of the insulin secretion from pancreas or a decrease of the insulin sensitivity in liver, skeletal muscle or adipose tissue. Peroxisome proliferator-activated receptor (PPAR) γ is a nuclear factor expressed in heart, muscle, colon, large intestine, kidney, pancreas, spleen, adipose tissue, and macrophage, which is activated by 15-deoxyprostaglandin J2 as a physiological ligand. It is known that PPAR γ is an essential transcription factor for adipocyte differentiation and also activates the glucose uptake in muscle. And, some thiazolidine derivatives are known as an antidiabetic drug, in particular an insulin-sensitizing agent which controls blood glucose by activating PPAR γ to increase the insulin sensitivity (Non-patent Reference 1).
Currently, hypoglycemic drugs and insulin are used as a drug for treating diabetes. The hypoglycemic drugs are classified into insulin secretagogues and non-insulin secretagogues. The insulin secretagogues include a sulfonylurea (SU) drug and a phenylalanine derivative which both stimulate the insulin secretion by binding to a SU receptor in pancreatic β cells, and also include a GLP-1 (glucagon like peptide-1) derivative and a GLP-1 degrading enzyme (dipeptidyl peptide protease-4, DPP-4) inhibitor which both enhance the insulin secretion by binding to a GLP-1 receptor in pancreatic β cells. On the other hand, the non-insulin secretagogues include a biguanide which has an action of inhibiting gluconeogenesis in liver, inhibiting absorption of glucose in gastrointestinal tract, or improving insulin sensitivity in peripheral tissue; and also include an α-glucosidase inhibitor which has an action of inhibiting absorption of glucose. In addition, the insulin-sensitizing drugs are classified as a non-insulin secretagogue.
According to the Japanese clinical practice guideline for diabetes, any of the currently used hypoglycemic drugs are perceived to show an improvement in glycemic control, and thus any drugs may be a first-line agent as long as they provide a good glycemic control. During the treatment with the first-line agent, however, it is recommended to add another hypoglycemic drug having a different action mechanism from the first-line agent, or switch to insulin or combine with insulin because blood glucose level would gradually be increased in many of the patients after treating with the single agent for a long period (i.e. secondary failure) though the single-agent therapy could achieve a good glycemic control in patients at the outset of the treatment. Furthermore, it is recommended to use the thiazolidine derivative in combination with a SU drug, a biguanide, or an α-glucosidase inhibitor (Non-patent Reference 2). On the other hand, it has not been reported that a combination of an antidiabetic drug and a heteroaryl derivative having PPAR γ transcriptional activity can be useful as a hypoglycemic agent though Patent Reference 1 discloses such heteroaryl derivative.
[Patent Reference 1] WO 2005/012245 A1
[Non-patent Reference 1] Joslin's Diabetes Mellitus 2nd Edition; Medical Sciences International, Ltd.; 2007; 769-794.
[Non-patent Reference 2] Clinical Guidelines for Treating Diabetes Based on Scientific Evidence; Nankodo; 2004; 37-46.

### DISCLOSURE OF INVENTION

### (Problems to Be Solved by Invention)

The purpose of the present invention is to provide a hypoglycemic agent useful for treating diabetes or other similar diseases without adverse effects.

### (Means to Solve the Problem)

The present inventors have extensively studied to find that hypoglycemic effects can be enhanced by using an antidiabetic agent in combination with a heteroaryl derivative having PPAR γ transcriptional activity, thus the combination is extremely useful as a utility medicine. Based upon the new findings, the present invention has been completed.

The present invention relates to the followings:
[1] A hypoglycemic agent comprising a combination of an antidiabetic drug and 2-methyl-2-[(4-{(1E)-3-[2-(4-methylbenzoyl)-1H-pyrrol-1-yl]prop-1-en-1-yl}benzyl)oxy]-propanoic acid or a pharmaceutically acceptable salt thereof.
[2] The hypoglycemic agent of [1] wherein the antidiabetic drug is an insulin secretagogue.
[3] The hypoglycemic agent of [1] wherein the antidiabetic drug is an insulin preparation.
[4] The hypoglycemic agent of [2] a wherein the insulin secretagogue is a SU drug.
[5] The hypoglycemic agent of [2] wherein the insulin secretagogue is a phenylalanine derivative.
[6] The hypoglycemic agent of [2] wherein the insulin secretagogue is a GLP-1 analogue.
[7] The hypoglycemic agent of [2] wherein the insulin secretagogue is a DPP-4 inhibitor.
[8] The hypoglycemic agent of [1] wherein the antidiabetic drug is a non-insulin secretagogue.
[9] The hypoglycemic agent of [8] wherein the non-insulin secretagogue is a biguanide;
[10] The hypoglycemic agent of [8] wherein the non-insulin secretagogue is an α-glucosidase inhibitor.

[11] A method for lowering blood glucose in a mammal comprising administering to the mammal an antidiabetic drug and 2-methyl-2-[(4-{(1E)-3-[2-(4-methylbenzoyl)-1H-pyrrol-1-yl]prop-1-en-1-yl}benzyl}oxy]propanoic acid or a pharmaceutically acceptable salt thereof.
[12] Use of an antidiabetic drug and 2-methyl-2-[(4-{(1E)-3-[2-(4-methylbenzoyl)-1H-pyrrol-1-yl]prop-1-en-1-yl}benzyl)oxy]propanoic acid or a pharmaceutically acceptable salt thereof for the manufacture of a hypoglycemic drug.

[13] A hypoglycemic agent comprising 2-ethyl-2-[(4-{(1E)-3-[2-(4-methylbenzoyl)-1H-pyrrol-1-yl]prop-1-en-1-yl}benzyl)oxy]propanoic acid or a pharmaceutically acceptable salt thereof for use in combination with a pharmaceutical composition comprising an antidiabetic drug.
[14] The hypoglycemic agent of [13] wherein the antidiabetic drug is an insulin secretagogue.
[15] The hypoglycemic agent of [14] wherein the insulin secretagogue is a SU drug.
[16] The hypoglycemic agent of [14] wherein the insulin secretagogue is a phenylalanine derivative.
[17] The hypoglycemic agent of [14] wherein the insulin secretagogue is a GLP-1 analogue.
[18] The hypoglycemic agent of [14] wherein the insulin secretagogue is a DPP-4 inhibitor.
[19] The hypoglycemic agent of [13] wherein the antidiabetic drug is a non-insulin secretagogue.
[20] The hypoglycemic agent of [19] wherein the non-insulin secretagogue is a biguanide.
[21] The hypoglycemic agent of [19] wherein the non-insulin secretagogue is an α-glucosidase inhibitor.

[22] A method for increasing the hypoglycemic effect of 2-methyl-2-[(4-{(1E)-3-[2-(4-methylbenzoyl)-1H-pyrrol-1-yl]prop-1-en-1-yl}benzyl)oxy]propanoic acid or a pharmaceutically acceptable salt thereof in a mammal comprising administering to the mammal an antidiabetic drug.

[23] An agent for increasing hypoglycemic effect of an antidiabetic drug comprising 2-methyl-2-[(4-{(1E)-3-[2-(4-methylbenzoyl)-1H-pyrrol-1-yl]prop-1-en-1-yl}benzyl)oxyl-propanoic acid or a pharmaceutically acceptable salt thereof.
[24] The agent of [23] wherein the antidiabetic drug is an insulin secretagogue.
[25] The agent of [24] wherein the insulin secretagogue is a SU drug.
[26] The agent of [24] wherein the insulin secretagogue is a phenylalanine derivative.
[27] The agent of [24] wherein the insulin secretagogue is a GLP-1 analogue.
[28] The agent of [24] wherein the insulin secretagogue is a DPP-4 inhibitor.
[29] The agent of [23] wherein the antidiabetic drug is a non-insulin secretagogue.
[30] The agent of [29] wherein the non-insulin secretagogue is a biguanide.
[31] The agent of [29] wherein the non-insulin secretagogue is an α-glucosidase inhibitor.

[32] A method for increasing the hypoglycemic effect of an antidiabetic drug in a mammal comprising administering to the mammal 2-methyl-2-[(4-{(1E)-3-[2-(4-methylbenzoyl)-1H-pyrrol-1-yl]prop-1-en-1-yl}benzyl)oxy]-propanoic acid or a pharmaceutically acceptable salt thereof.

[33] Use of 2-methyl-2-[(4-{(1E)-3-[2-(4-methylbenzoyl)-1H-pyrrol-1-yl]prop-1-en-1-yl}benzyl)oxy]propanoic acid or a pharmaceutically acceptable salt thereof for the manufacture of an agent for increasing hypoglycemic effect of an antidiabetic drug; or

[34] A method for lowering blood glucose in a mammal comprising administering to the mammal an antidiabetic drug and 2-methyl-2-[(4-{(1E)-3-[2-(4-methylbenzoyl)-1H-pyrrol-1-yl]prop-1-en-1-yl}benzyl)oxy]propanoic acid or a pharmaceutically acceptable salt thereof, which lowers blood glucose more effectively than when each of the said drugs is administered alone.

### (Effect of Invention)

The hypoglycemic agent of the present invention exhibits an excellent effect of controlling hyperglycemia, and thereby it is useful for treating diabetes or other similar diseases.
The hypoglycemic agent of the present invention exhibits an excellent hypoglycemic effect in diabetic patients, and thereby the agent can also inhibit the development of diabetic complications (e.g. diabetic neuropathy, diabetic nephropathy, diabetic retinopathy, and arteriosclerosis) from diabetes.
In addition, the hypoglycemic agent of the present invention can reduce adverse effects of an antidiabetic drug because the present agent can maintain hypoglycemic effects even if the dose of the antidiabetic drug is lowered. The adverse effects include, for example, vascular complications and hypoglycemia which is caused by insulin preparation; and hypoglycemia and pancreas exhaustion which is caused by insulin secretagogue. Consequently, the hypoglycemic agent of the present invention can be safely administered for a prolonged period to a patient suffering from diabetes or other similar diseases.

### BEST MODE FOR CARRYING OUT INVENTION

In the following, the present invention is described in more detail.

The antidiabetic drug of the present invention is defined as a compound which lowers blood glucose, and the compound may be either peptidic or nonpeptidic compound. In addition, as long as the antidiabetic drug exerts its activity, the form of the drug in vivo after administration may be different from that of the drug before administration. Namely, the antidiabetic drug may be an "active metabolite" which exhibits an antidiabetic activity after its structure has changed by metabolism in vivo. Furthermore, the antidiabetic drug may be a "prodrug" which can change into an active form through a reaction with an enzyme or gastric acid under physiological conditions in vivo.
The antidiabetic drug may include, for example, a hypoglycemic drug, an insulin preparation, and other drugs.
Two or more of these drugs may be used together in an appropriate ratio.
The insulin secretagogue, one of the hypoglycemic drugs, includes, for example, a SU drug, a phenylalanine derivative, a GLP-1 analogue, and a DPP-4 inhibitor.
The SU drug includes, for example, glibenclamide, gliclazide, glipizide, and glimepiride; and the phenylalanine derivative includes, for example, repaglinide, nateglinide, and mitiglinide.
The GLP-1 analogue includes, for example, liraglutide, exenatide, Inslinotropin (GLP-1), Albiglutide (GSK-716155), CJC-1131 (DAC;GLP-1), AVE-0010 (ZP-10, ZP-10A), BIM-51077 (ITM-077, R-1583), GLP1-INT (TT-233/GLP1), PC-DAC: Exendin-4 (CJC-1134-PC), and LY-2189265; and the DPP-4 inhibitor includes, for example, Sitagliptin, Vildagliptin, Alogliptin, Saxagliptin, Denagliptin, Melogliptin, Linagliptin, ALS-2-0426, KRP-104, MP-513, P93/01, PF-734200, PHX-1149, R-1579, SK-0403, SYR-472, T-6666, ABT-279, TAK-100, ARI-2243, A-916165, and DSP-7238.
On the other hand, the non-insulin secretagogue, one of the hypoglycemic drugs, includes, for example, a biguanide and an α-glucosidase inhibitor.
The biguanide includes, for example, metformin or a salt thereof, buformin or a salt thereof, and phenformin or a salt thereof. Metformin hydrochloride is preferred.
The α-glucosidase inhibitor includes, for example, voglibose, acarbose, miglitol, and emiglitate.
In addition, the followings are specific examples of compounds which lower blood glucose: a sodium-dependent glucose transporter (SGLT) inhibitor, an 11β-hydroxysteroid dehydrogenase inhibitor, a glucokinase activator, a glucagon receptor antagonist, a fructose 1,6-bisphosphatase inhibitor, and a glycogen phosphorylase inhibitor.
The SGLT inhibitor includes, for example, Dapagliflozin, Sergliflozin, Remogliflozin, AVE-2268, GSK-189075, ASP-1941, YM-543, KGT1075, TA7284, CDG - 452 (R-7201), SAR-7226, TS-033, T1095, BI 10773, and BI 44847. The 11β-hydroxysteroid dehydrogenase inhibitor includes, for example, PF-915275 and INCB123739; and the glucokinase activator includes, for example, R1551, AZD6370, LY2599506, TTP355, AMG-221, and PSN-010. The glucagon receptor antagonist includes, for example, BAY27-9955 and NNC25-2504. The fructose 1,6-bisphosphatase inhibitor includes, for example, MBX-07803. The glycogen phosphorylase inhibitor includes, for example, PSN-357.

The salt used herein includes, for example, a salt formed with inorganic acids such as hydrochloride, hydrobromide, hydroiodide, sulfate, and nitrate; and a salt formed with organic acids such as acetate, oxalate, citrate, malate, tartrate, fumarate, maleate, methanesulfonate, and benzenesulfonate.
Furthermore, the salt also includes, for example, a salt formed with organic bases such as diethanolamine salt, ethylenediamine salt, or N-methylglucamine salt; a salt formed with alkaline earth metals such as calcium salt or magnesium salt; and a salt formed with alkali metals such as lithium salt, potassium salt, or sodium salt.

The antidiabetic drug and other similar drugs used herein may be an anhydride or solvate (e.g. hydrate) thereof.

The structure of 2-methyl-2-[(4-{(1E)-3-[2-(4-methylbenzoyl)-1H-pyrrol-1-yl]prop-1-en-1-yl}benzyl)oxy]-propanoic acid is shown below (hereinafter, referred to as "Compound A").

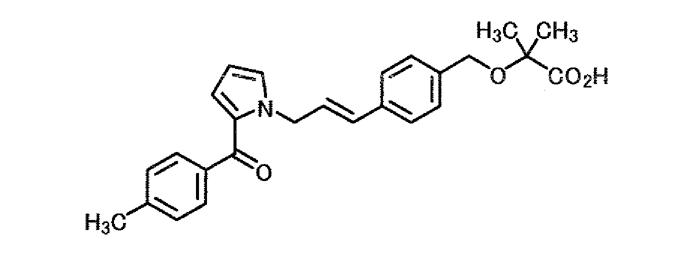

The hypoglycemic agent of the present invention comprises a combination of (A) an antidiabetic drug or other similar drugs and (B) Compound A or other similar compounds, namely, the present invention may include any combinations of (A) an antidiabetic drug or other similar drugs and (B) Compound A or other similar compounds as long as (A) and (B) are administered in a combination. Thus, as long as the pharmaceutical composition of the present invention is administrated in a combination of (A) an antidiabetic drug or other similar drugs and (B) Compound A or other similar compounds; (A) and (B) may be formulated in a single unit formulation or separately formulated in two unit formulations.
Examples of the dosage forms include, but are not limited to the followings:
(a) administration of a composition comprising (A) an antidiabetic drug or other similar drugs and (B) Compound A or other similar compounds, i.e. administered in a single unit formulation;
(b) coadministration of two formulations in the same administration route wherein one of the formulation is formulated from (A) an antidiabetic drug or other similar drugs and the other one is formulated from (B) Compound A or other similar compounds;
(c) administration of two formulations with a time lag in the same administration route wherein one of the formulation is formulated from (A) an antidiabetic drug or other similar drugs and the other one is formulated from (B) Compound A or other similar compounds (e.g. administering (A) an antidiabetic drug or other similar drugs and then (B) Compound A or other similar compounds, or administering them in the reverse order);
(d) coadministration of two formulations in a different administration route wherein one of the formulation is formulated from (A) an antidiabetic drug or other similar drugs and the other one is formulated from (B) Compound A or other similar compounds; and
(e) administration of two formulations with a time lag in a different administration route wherein one of the formulation is formulated from (A) an antidiabetic drug or other similar drugs and the other one is formulated from (B) Compound A or other similar compounds (e.g. administering (A) the antidiabetic drug or other similar drugs and then (B) Compound A or other similar compounds, or administering them in the reverse order).
In addition, when the above-mentioned two formulations are administered with a time lag, it is necessary for both (A) an antidiabetic drug or other similar drugs and (B) Compound A or other similar compounds to exist together in vivo for a certain period of time so that it would be enough to enhance the hypoglycemic effect and blood insulin elevating effect.

The present invention includes a commercial package comprising a combination drug comprising (A) an antidiabetic drug or other similar drugs and (B) Compound A or other similar compounds, and a package insert informing that the combination drug can or should be used to enhance the hypoglycemic effect and blood insulin elevating effect; a commercial package comprising an antidiabetic drug or other similar drugs, and a package insert informing that the pharmaceutical composition can or should be used to enhance the hypoglycemic effect and blood insulin elevating effect of Compound A or other similar compounds; and a commercial package comprising Compound A or other similar compounds, and a package insert informing that the pharmaceutical composition can or should be used to enhance the hypoglycemic effect and blood insulin elevating effect of an antidiabetic drug or other similar compounds.

The hypoglycemic agent of the present invention is useful for controlling postprandial hyperglycemia in a prediabetic state; preventing and treating non-insulin-dependent diabetes mellitus; preventing and treating diabetic complication; treating autoimmune disease such as rheumatoid arthritis and multiple sclerosis; treating intestinal mucosal disease such as ulcerative colitis and Crohn's disease; treating hepatic cirrhosis; treating chronic respiratory disease such as asthma, chronic obstructive pulmonary disease, and pulmonary fibrosis; treating Parkinson's disease; treating obesity; and treating cancer.

When the hypoglycemic agent of the present invention is clinically used, the present agent can be administered orally or parenterally (e.g. intravenously, subcutaneously, intramuscularly, topically, transrectally, transcutaneously, or nasally). The compositions for oral administration include, for example, tablets, capsules, pills, granules, powders, liquids, and suspensions; and the compositions for parenteral administration include, for example, injectable aqueous or oily solutions, ointments, creams, lotions, aerosols, suppositories, and adhesive skin patches. The above-exemplified formulation can be prepared by conventional techniques, and the formulations may also comprise nontoxic and inert carriers or additive agents which are commonly used in the medicinal field.

The dose of the hypoglycemic agent of the present invention can be varied corresponding to the property of the compound and patient's disease, age, body weight, sex, symptom, administration route, or other factors. Usually, an antidiabetic drug and Compound A are each administered in a dose of 0.01-3000 mg/day, preferably 0.1-2550 mg/day to an adult (body weight 50 kg), which can be administered once per day or in two to three times per day with the divided doses. In addition, the frequency may be varied from once in several days to once in several weeks.

### EXAMPLE

The present invention is illustrated in more detail by the following reference examples, examples, and tests, but it should not be construed to be limited thereto. In addition, the compound names of the following examples do not necessarily correspond to IUPAC Names. Furthermore, some terms are defined by abbreviations for the sake of shorthand, which are as defined above.

### Example 1-1

The antidiabetic action resulting from the combination of an insulin secretagogue (SU drug) and Compound A was studied by using C57BL/KsJ-db/db mice (hereinafter, referred to as "db/db mice") (9 weeks old, male, CLEA Japan, Inc.) as a model of type 2 diabetes. The db/db mice were allocated to 4 groups (8 mice/group), and each group was put into a cage provided with feed. Among the 4 groups, a vehicle (0.5 % methylcellulose solution) was administered to Groups 1 and 2 by gavage once a day for 15 days, and Compound A (30 mg/kg) was administered to Groups 3 and 4 under the same condition as Groups 1 and 2. On the next day after the last administration, the vehicle was administered to Groups 1 and 3, and glibenclamide (10 mg/kg) was administered to Groups 2 and 4, both by gavage; then the feed were removed from the cages and the changes in blood glucose levels were studied in each group.

Table 1 shows the changes in blood glucose levels and Table 2 shows the areas under the curve of blood glucose level, wherein the results were obtained from the mice which were repeatedly administered with the vehicle or Compound A once a day for 15 days and then administered with the vehicle or glibenclamide on the next day after the last administration. As for Groups 1 and 2 (i.e. repeatedly administered with the vehicle), there was no decrease in blood glucose levels in both the groups with and without glibenclamide. However, as for Groups 3 and 4 (i.e. repeatedly administered with Compound A), decrease of blood glucose levels were observed in the group with glibenclamide whereas the group without glibenclamide did not, which means that the hypoglycemic effect of Compound A is enhanced by combining glibenclamide. Table 3 shows the changes in blood insulin and Table 4 shows the areas under the curve of blood insulin level, which were obtained after the administration of glibenclamide. As a result, blood insulin level was higher in Group 4 (i.e. repeatedly administered with Compound A and then with glibenclamide) than in Groups 1 and 2 (i.e. repeatedly administered with the vehicle).

**[Table 1]**

| Group | Repeatedly administered agent | Glibenclamide administration | Blood glucose level before the glibenclamide administration (mg/dL) | Blood glucose level after the glibenclamide administration (%, relative value when blood glucose level at 0 hr is 100 %) | | | |
|---|---|---|---|---|---|---|---|
| | | | | 1 hr | 2 hr | 4 hr | 6 hr |
| 1 | Vehicle | - | 402.6±40.9 | 94.9±10.4 | 95.0± 8.5 | 86.8± 8.6 | 88.9±7.5 |
| 2 | Vehicle | + | 422.0±36.4 | 96.2±7.7 | 91.3±5.9 | 82.9±12.7 | 80.9±12.1 |
| 3 | Compound A | - | 332.1±90.8 | 100.2±10.2 | 96.1±14.2 | 93.8±15.9 | 93.6±14.1 |
| 4 | Compound A | + | 323.4±75.7 | 91.1±11.2 | 82.8±11.8 | 75.2*±17.2 | 79.0±18.1 |

| | | | | | | | |
|---|---|---|---|---|---|---|---|
| Mean value ± standard deviation (n=8) *P<0.05 Group 3 vs Group 4 (Student's t-test, significance level, two sided 5 %) | | | | | | | |

There was no significant difference between Group 1 and Group 2 (Student's t-test, significance level, two sided 5 %).

**[Table 2]**

| Group | Repeatedly administered agent | Glibenclamide administration | Area under the curve of blood glucose level after the glibenclamide administration (%·hr, relative value when blood glucose level at 0 hr is 100 %) |
|---|---|---|---|
| 1 | Vehicle | - | 550.0±41.1 |
| 2 | Vehicle | + | 530.0±35.1 |
| 3 | Compound A | - | 575.6±73.0 |
| 4 | Compound A | + | 494.7±74.8* |

| | | | |
|---|---|---|---|
| Mean value ± standard deviation (n=8) *P<0.05 Group 3 vs Group 4 (Student's t-test, significance level, two sided 5 %) | | | |

There was no significant difference between Group 1 and Group 2 (Student's t-test, significance level, two sided 5 %).

**[Table 3]**

| Group | Repeatedly administered agent | Glibenclamide administration | Blood glucose level before the glibenclamide administration (mg/dL) | Blood insulin level after the glibenclamide administration (%, relative value when insulin level at 0 hr is 100 %) | |
|---|---|---|---|---|---|
| | | | | 1 hr | 2 hr |
| 1 | Vehicle | - | 23.2±11.3 | 65.8±12.6 | 85.9±53.2 |
| 2 | Vehicle | + | 18.7± 8.0 | 75.1±25.1 | 83.3±21.4 |
| 3 | Compound A | - | 25.4±13.8 | 55.9±15.8 | 61.6±17.3 |
| 4 | Compound A | + | 33.2±20.8 | 100.3±19.5** | 82.2+11.6* |

| | | | | | |
|---|---|---|---|---|---|
| Mean value ± standard deviation (n=8) *<0.05, **P<0.01 Group 3 vs Group 4 (Student's t-test, significance level, two sided 5 %) | | | | | |

There was no significant difference between Group 1 and Group 2 (Student's t-test, significance level, two sided 5 %).

**[Table 4]**

| Group | Repeatedly administered agent | Glibenclamide administration | Area under the curve of blood insulin level after the glibenclamide administration (%·hr, relative value when insulin level at 0 hr is 100 %) |
|---|---|---|---|
| 1 | Vehicle | - | 158.7±23.5 |
| 2 | Vehicle | + | 166.7±31.5 |
| 3 | Compound A | - | 136.8±17.7 |
| 4 | Compound A | + | 191.3±20.5 ** |

| | | | |
|---|---|---|---|
| Mean value ± standard deviation (n=8) **P<0.01 Group 3 vs Group 4 (Student's t-test, significance level, two sided 5 %) | | | |

There was no significant difference between Group 1 and Group 2 (Student's t-test, significance level, two sided 5 %).

### Example 1-2

The antidiabetic action resulting from the combination of an insulin secretagogue (DPP-4 inhibitor) and Compound A was studied by using *db*/*db* mice (8 weeks old, male, CLEA Japan, Inc.). The db/db mice were allocated to 4 groups (8 mice/group). The mice in Group 1 were given powdered feed (control), Group 2 were given powdered feed containing 0.02 (w/w) % of Compound A, Group 3 were given powdered feed containing 0.06 (w/w) % of sitagliptin, and Group 4 were given powdered feed containing 0.02 (w/w) % of Compound A and 0.06 (w/w) % of sitagliptin. In addition, the powdered feed mentioned-above were all the same (i.e. trade name: CE-2, CLEA Japan, Inc.). An oral glucose tolerance test was performed on Day 28. To be more specific, on the day before the test, the mice were fasted overnight; and on the test day, 2 g/kg of glucose was administered by gavage to study the changes in blood glucose levels. Glycosylated hemoglobin (hereinafter, referred to as "HbA1c") was measured on Day 33.

Table 5 shows the changes in blood glucose levels and Table 6 shows the areas under the curve of blood glucose level, which were obtained from the oral glucose tolerance test performed on Day 28. A significant difference was not observed between the group given Compound A or sitagliptin alone and the control, but in the group where the two agents were combined, the area under the curve of blood glucose level was decreased, i.e. a hypoglycemic effect was observed. Table 7 shows the amounts of change in HbA1c from the start of the experiment to Day 33. A difference was not observed between the group given Compound A or sitagliptin alone and the control, but in the group where the two agents were combined, a synergistic effect of lowering HbAlc was observed.

**[Table 5]**

| Group | Treatment | Blood glucose level after the glucose load on Day 28 (mg/dL) | | | | | |
|---|---|---|---|---|---|---|---|
| | | 0 min | 10 min | 30min | 60 min | 90min | 120 min |
| 1 | Control | 153±25 | 546± 83 | 654±70 | 586±51 | 522±37 | 486±64 |
| 2 | Compound A | 146±33 | 500±62 | 633±57 | 566±41 | 457±74 | 412±82 |
| 3 | Sitagliptin | 167±15 | 542±81 | 588±125 | 510±95 | 455±100 | 408±107 |
| 4 | Compound A + Sitagliptin | 132^{*,†}±21 | 436^{*,†}±77 | 458^{**,##,††}±86 | 383^{**,##,††} ±104 | 345^{**,##,†}±57 | 273^{**,##,††}±71 |

| | | | | | | | |
|---|---|---|---|---|---|---|---|
| Mean value ± standard deviation (=8) *P<0.05, **P<0.01 vs Control, ##P<0.01 vs Compound A, †P<0.05, ††P<0.01 vs sitagliptin (Student's t-test) | | | | | | | |

**[Table 6]**

| Group | Treatment | Area under the curve of blood glucose level after the glucose load on Day 28 (mg·min/dL) |
|---|---|---|
| 1 | Control | 64572±5158 |
| 2 | Compound A | 59692±4741 |
| 3 | Sitagliptin | 57680±10679 |
| 4 | Compound. A + sitagliptin | 43786±8355^{**, ##, $$} |

| | | |
|---|---|---|
| Mean value ± standard deviation (n=8) **P<0.01 vs Control, ##P<0.01 vs Compound A, ^{$$}p<0.01 vs sitagliptin (Student's t-test) | | |

**[Table 7]**

| Group | Treatment | Amounts of change in HbA1c before and after treatment (%) |
|---|---|---|
| 1 | Control | 2.05-0.81 |
| 2 | Compound A | 1.28±0.53 |
| 3 | Sitagliptin | 1.68±0.51 |
| 4 | Compound A + Sitagliptin | 0.91±0. 65 ^{**} |

| | | |
|---|---|---|
| Mean value ± standard deviation (n=8) **P<0.01 vs Control (Dunnett's t-test) | | |

### Example 1-3

The antidiabetic action resulting from the combination of an insulin-sensitizing drug and a biguanide was studied by using db/db mice (8 weeks old, male, CLEA Japan, Inc.). The db/db mice were allocated to 4 groups (8 mice/group). Among the 4 groups, a vehicle (0.5 % methylcellulose solution) was administered to Group 1 by gavage once a day for 14 days from the day of grouping (Day 1); Compound A (30 mg/kg) was administered to Group 2, metformin (300 mg/kg) was administered to Group 3, and Compound A (30 mg/kg) and metformin (300 mg/kg) were administered to Group 4 under the same condition as Group 1. Blood glucose and HbAlc levels were measured on Day 11 and Day 15, respectively.

Table 8 shows the blood glucose levels of Day 11 and Table 9 shows the amounts of change in HbA1c from Day 1 to Day 15. As a result, a significant decrease of blood glucose level and amount of HbAlc change were observed in Groups 2 and 3 (i.e. administered with Compound A or metformin alone), compared with Group 1 (i.e. administered with the vehicle), which means that a hypoglycemic effect was observed. Furthermore, a significant decrease of blood glucose level and amount of HbAlc change were observed in Group 4 (i.e. administered with Compound A and metformin together), compared with Groups 2 and 3 (i.e. administered with Compound A or metformin alone), which means that a synergistic hypoglycemic-effect was observed.

**[Table 8]**

| Group | Treatment | Blood glucose level |
|---|---|---|
| 1 | Vehicle | 418.3 ± 39.7 |
| 2 | Compound A | 321.9 ± 43.5 ** |
| 3 | Metformin | 323.5 ± 66.3 ** |
| 4 | Compound A + Metformin | 236.3 ± 60.7 **^{,#,†} |

| | | |
|---|---|---|
| Mean value ± standard deviation (n=8) **P<0.01 vs Vehicle, #P<0.05 vs Compound A, †P<0.05 vs metformin (Turkey's test) | | |

**[Table 9]**

| Group | Treatment | Amount of change in HbA1c |
|---|---|---|
| 1 | Vehicle | 1.58 ± 0.18 |
| 2 | Compound A | 0.93 ± 0.28** |
| 3 | Metformin | 1.09 ± 0.16 ** |
| 4 | Compound A + metformin | 0.48 ± 0.25 **^{,##,††} |

| | | |
|---|---|---|
| **P<0.01 vs Vehicle, ##P<0.01 vs Compound A, ††P<0.01 vs metformin (Turkey's test) | | |

### Example 2

Synthesis of 2-methyl-2-[(4-{(1E)-3-[2-(4-methylbenzoyl)-1H-pyrrol-1-yl]prop-1-en-1-yl}benzyl)oxy]propanoic acid

### Example 2-1

### (4-Methylphenyl)(1H-pyrrol-2-yl)methanone

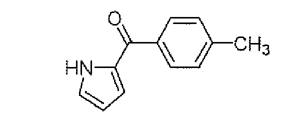

Ethyl bromide (28.0 g, 257 mmol) was added dropwise to a suspension of magnesium (17.6 g, 114 mmol) in THF. Then, pyrrole (15.3 g, 228 mmol) was added thereto, and then a solution of p-toluoyl chloride (17.6 g, 114 mmol) in toluene was added dropwise thereto. The reaction mixture was added to 3 % aqueous hydrochloric acid, then the resultant solution was adjusted to neutral with aqueous hydrochloric acid and the mixture was partitioned with a separating funnel. The organic layer was washed with water, concentrated, and crystallized from toluene/n-heptane. The precipitated crystal was collected through a filter and dried to give the title compound (20.7 g, 83 %). ¹H NMR (CDCl₃, 400 MHz) δ9. 87 (brs, 1H) , 7.83 (d, 2 H, J = 8.2 Hz), 7.29 (d, 2 H, J = 8.2 Hz), 7.15-7.13 (m, 1H), 6.91- 6.88 (m, 1H), 6.35- 6.32 (m, 1H), 2.44 (s, 3 H).

### Example 2-2

### (1-Allyl-1H-pyrrole-2-yl) (4-methylphenyl)methanone

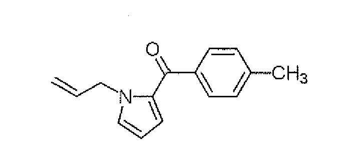

To a solution of potassium tert-butoxide (20.0 g, 178 mmol) in THF, a solution of the compound of Example 2-1 (30.0 g, 162 mmol) in THF was added, and then allyl bromide (1.62 g, 13.4 mmol) was added dropwise. After the reaction was completed, the mixture was extracted with toluene and brine. The organic layer was washed with brine and then concentrated to prepare the title compound (37.4 g, quantitative) .
¹H NMR (CDCl₃, 400 MHz) δ 7.71 (d, 2H, J = 8.1Hz), 7.25 (d, 2 H, J = 8.1Hz), 6.98 (dd, 1H, J = 2.5, 1.6 Hz), 6.74 (dd, 1H, J = 4.0, 1.6 Hz), 6.19 (dd, 1H, J = 4.0, 2.5 Hz), 6.07 (ddt, 1H, J = 16.7, 10.3, 5.6 Hz), 5.16 (dq, 1H, J = 10.3, 1.3 Hz), 5.07 (dq, 1H, J = 16.7, 1.3 Hz), 5.05 (dt, 2 H, J = 5.6, 1.3 Hz), 2.42 (s, 3H).

### Example 2-3

### 4-Iodobenzyl bromide

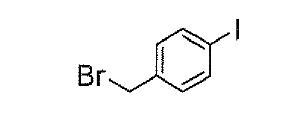

To a solution of 4-iodotoluene (10.0 g, 45.9 mmol) in dichloromethane (70 ml), bromine (3.6 ml, 69.9 mmol) and a solution of 30 % hydrogen peroxide (5.2 g, 45.9 mmol) in water (70 ml) were added in order at room temperature. The reaction solution was heated, and vigorously stirred for 10 hours under reflux (bath temperature: 50 °C).
The reaction solution was transferred to a separating funnel, chloroform (40 ml) and water (20 ml) was added to the funnel to separate layers, and the organic layer was washed with water (150 ml) three times. The organic layer was washed with 0.5 % aqueous sodium bisulfite (150 ml) and then water (150 ml), and the solvent was removed under reduced pressure (bath temperature: 25 °C). Before completely removing the solvent, toluene (50 ml) was added to the mixture, then the mixture was concentrated, and this process was performed twice. The mixture was concentrated to dryness, and the residue was dried under vacuum to prepare iodobenzyl bromide (12.1 g).
¹H NMR (CDCl₃, 400 MHz) δ7.68 (d, 2H, J = 8.3 Hz), 7.13 (d, 2H, J = 8.3 Hz), 4.23 (s, 2 H)

### Example 2-4

### 2-[(4-4-Iodobenzyl)oxy]-2-methylpropionic acid

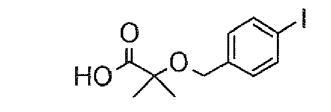

A solution of methyl 2-hydroxyisobutyrate (9.95 g, 84.2 mmol) in THF was added dropwise to a suspension of sodium hydride (58.8 % in parafin liquid) (3.30 g, 80.8 mmol) in THF, and the temperature was maintained at room temperature. The reaction solution was added dropwise to a solution of 4-iodobenzylbromide (20.0 g, 67.4 mmol) in THF which was maintained at 60 °C. After the reaction was completed, the mixture was cooled to 35 °C, and methanol and 13 % aqueous potassium hydroxide solution were added to the mixture, and then the mixture was maintained at the temperature. The mixture was extracted with toluene, then the aqueous layer was re-extracted by adding aqueous hydrochloric acid and toluene, and the organic layer was further washed with water. The combined organic layer was concentrated and crystallized from toluene/n-heptane. The precipitated crystal was collected through a filter and dried to prepare the title compound (17.2 g, 80 %).
¹H NMR (CDCl₃, 400 MHz) δ7.67 (d, 2H, J = 8.3 Hz), 7.13 (d, 2H, J = 8.3 Hz), 4.47 (s, 2H), 1.55 (s, 6H)

### Example 2-5

### 2-Methyl-2-[(4-{(1E)-3-[2-(4-methylbenzoyl)-1H-pyrrol-1-yl]prop-1-en-1-yl}benzyl)oxy]propanoic acid

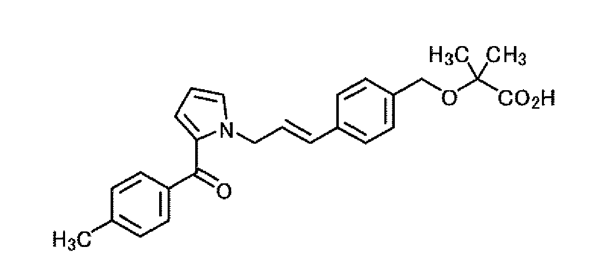

10 % Palladium carbon (1.40 g, 0.656 mmol, containing 50 % water) was added to a suspension of Example 2-4 (14.0 g, 43.7 mmol), a solution of Example 2-2 in toluene (20.4 g, 53.2 %, 48.1 mmol), benzyltriethyl ammonium chloride (10.0 g, 43.7 mmol) and dicyclohexylmethylamine (12.8 g, 65.6 mmol) in toluene, and the temperature was maintained at 60 °C. After the reaction was completed, toluene and THF was added to the mixture, and the mixture was filtrated. To the filtrate was added 10 % aqueous potassium hydroxide solution and THF, and the mixture was partitioned into two layers. The aqueous layer was acidified with aqueous hydrochloric acid, and extracted with toluene, and the toluene layer was further washed with water. The combined organic layer was treated with active carbon and then concentrated, and the concentrated residue was crystallized from toluene/n-heptane. The precipitated crystal was collected through a filter, and the resultant crystal was recrystallized from acetone/water to prepare the title compound (14.0 g, 78 %).
¹H NMR (CDCl₃, 400 MHz) δ 7.73 (d, 2H, J = 8.0 Hz), 7.40-7.20 (m, 6H), 7.05 (dd, 1H, J = 2.4, 1.7 Hz), 6.77 (dd, 1H, J = 4.0, 1.7 Hz), 6.51 (d, 1H, J = 16.0Hz) , 6.45 (dt, 1H, J = 16.0, 5.0Hz), 6.21 (dd, 1H, J = 4.0, 2.4 Hz), 5.20 (d, 2H, J = 5.0 Hz), 4.49 (s, 2H), 2.42 (s, 3H), 1.56 (s, 6H),
MS(ESI): m/z 418 (M+1)

### Example 3

The following components 1 - 5 are mixed, then wet granulated using a water solution of component 6, and further mixed with component 7. The resultant mixture is compressed into a tablet (120 mg).

| | | |
|---|---|---|
| 1. | Compound A | 10 mg per tablet |
| 2. | Glibenclamide | 5 mg per tablet |
| 3. | Lactose | 67 mg per tablet |
| 4. | Corn starch | 30 mg per tablet |
| 5. | Carboxymethylcellulose calcium | 5 mg per tablet |
| 6. | Hydroxypropylcellulose (HPC-L) | 2 mg per tablet |
| 7. | Magnesium stearate | 1 mg per tablet |

### Example 4

The following components 1 - 5 are mixed, then wet granulated using a water solution of component 6, and further mixed with component 7. The resultant mixture is compressed into a tablet (135 mg).

| | | |
|---|---|---|
| 1. | Compound A | 10 mg per tablet |
| 2. | Sitagliptin | 50 mg per tablet |
| 3. | Lactose | 37 mg per tablet |
| 4. | Corn starch | 30 mg per tablet |
| 5. | Carboxymethylcellulose calcium | 5 mg per tablet |
| 6. | Hydroxypropylcellulose (HPC-L) | 2 mg per tablet |
| 7. | Magnesium stearate | 1 mg per tablet |

### Example 5

The following components 1 - 5 are mixed, then wet granulated using a water solution of component 6, and further mixed with component 7. The resultant mixture is compressed into a tablet (620 mg).

| | | |
|---|---|---|
| 1. | Compound A | 10 mg per tablet |
| 2. | Metformin | 500 mg per tablet |
| 3. | Lactose | 40 mg per tablet |
| 4. | Corn starch | 30 mg per tablet |
| 5. | Carboxymethylcellulose calcium | 25 mg per tablet |
| 6. | Hydroxypropylcellulose (HPC-L) | 10 mg per tablet |
| 7. | Magnesium stearate | 5 mg per tablet |

### Example 6

(1) The following components 1 - 4 are mixed, then wet granulated using a water solution of component 5, and further mixed with component 6. The resultant mixture is compressed into a tablet (120 mg).

| | | |
|---|---|---|
| 1. | Compound A | 10 mg per tablet |
| 2. | Lactose | 72 mg per tablet |
| 3. | Corn starch | 30 mg per tablet |
| 4. | Carboxymethylcellulose calcium | 5 mg per tablet |
| 5. | Hydroxypropylcellulose (HPC-L) | 2 mg per tablet |
| 6. | Magnesium stearate | 1 mg per tablet |

(2) The tablet of (1) and any one of glibenclamide (5 mg), sitagliptin (50 mg), or metformin (500 mg) are simultaneously administered to diabetic patients.

### INDUSTRIAL APPLICABILITY

The hypoglycemic agent of the present invention exhibits an excellent effect of controlling hyperglycemia, and thereby it is useful for treating diabetes or other similar diseases.
The hypoglycemic agent of the present invention exhibits an excellent hypoglycemic effect in diabetic patients, and thereby the agent can also inhibit the progression of diabetic complications (e.g. diabetic neuropathy, diabetic nephropathy, diabetic retinopathy, and arteriosclerosis) from diabetes.
In addition, the hypoglycemic agent of the present invention can reduce adverse effects of an antidiabetic drug because the present agent can maintain hypoglycemic effects even if the dose of the antidiabetic drug is lowered. The adverse effects include, for example, vascular complications, hypoglycemia, or other similar diseases in an insulin preparation; hypoglycemia, pancreas exhaustion, or other similar diseases in an insulin secretagogue; an increase in body weight or body fat, edema due to an increase in circulating plasma volume, heart failure, or other similar diseases in an insulin-sensitizing agent; lactic acidosis or other similar diseases in a biguanide; and gastrointestinal dysfunction or other similar diseases in an α-glucosidase inhibitor. Consequently, the hypoglycemic agent of the present invention can be safely administered for a prolonged period of time to a patient affected with diabetes or other similar diseases.

## Claims

1. A hypoglycemic agent comprising a combination of an antidiabetic drug and 2-methyl-2-[(4-{(1E)-3-[2-(4-methylbenzoyl)-1H-pyrrol-1-yl]prop-1-en-1-yl}benzyl)oxy]propanoic acid or a pharmaceutically acceptable salt thereof.

2. The hypoglycemic agent of claim 1 wherein the antidiabetic drug is an insulin secretagogue.

3. The hypoglycemic agent of claim 1 wherein the antidiabetic drug is an insulin preparation.

4. The hypoglycemic agent of claim 2 wherein the insulin secretagogue is a SU drug.

5. The hypoglycemic agent of claim 2 wherein the insulin secretagogue is a phenylalanine derivative.

6. The hypoglycemic agent of claim 2 wherein the insulin secretagogue is a GLP-1 analogue.

7. The hypoglycemic agent of claim 2 wherein the insulin secretagogue is a DPP-4 inhibitor.

8. The hypoglycemic agent of claim 1 wherein the antidiabetic drug is a non-insulin secretagogue.

9. The hypoglycemic agent of claim 8 wherein the non-insulin secretagogue is a biguanide.

10. The hypoglycemic agent of claim 8 wherein the non-insulin secretagogue is an α-glucosidase inhibitor.

11. A method for lowering blood glucose in a mammal comprising administering to the mammal an antidiabetic drug and 2-methyl-2-[(4-{(1E)-3-[2-(4-methylbenzoyl)-1H-pyrrol-1-yl]prop-1-en-1-yl}benzyl)oxy]propanoic acid or a pharmaceutically acceptable salt thereof.

12. Use of an antidiabetic drug and 2-methyl-2-[(4-{(1E)-3-[2-(4-methylbenzoyl)-1H-pyrrol-1-yl]prop-1-en-1-yl}benzyl)oxy]propanoic acid or a pharmaceutically acceptable salt thereof for the manufacture of a hypoglycemic drug.

13. A hypoglycemic agent comprising 2-methyl-2-[(4-{(1E) - 3-[2-(4-methylbenzoyl)-1H-pyrrol-1-yl]prop-1-en-1-yl}benzyl)oxy]propanoic acid or a pharmaceutically acceptable salt thereof for use in combination with a pharmaceutical composition comprising an antidiabetic drug.

14. The hypoglycemic agent of claim 13 wherein the antidiabetic drug is an insulin secretagogue.

15. The hypoglycemic agent of claim 14 wherein the insulin secretagogue is a SU drug.

16. The hypoglycemic agent of claim 14 wherein the insulin secretagogue is a phenylalanine derivative.

17. The hypoglycemic agent of claim 14 wherein the insulin secretagogue is a GLP-1 analogue.

18. The hypoglycemic agent of claim 14 wherein the insulin secretagogue is a DPP-4 inhibitor.

19. The hypoglycemic agent of claim 13 wherein the antidiabetic drug is a non-insulin secretagogue.

20. The hypoglycemic agent of claim 19 wherein the non-insulin secretagogue is a biguanide.

21. The hypoglycemic agent of claim 19 wherein the non-insulin secretagogue is an α-glucosidase inhibitor.

22. A method for increasing the hypoglycemic effect of 2-methyl-2-[(4-{(1E)-3-[2-(4-methylbenzoyl)-1H-pyrrol-1-yl]prop-1-en-1-yl}benzyl)oxy]propanoic acid or a pharmaceutically acceptable salt thereof in a mammal comprising administering to the mammal an antidiabetic drug.

23. An agent for increasing hypoglycemic effect of an antidiabetic drug comprising 2-methyl-2-[(4-{(1E)-3-[2-(4-methylbenzoyl)-1H-pyrrol-1-yl]prop-1-en-1-yl}benzyl)oxy]propanoic acid or a pharmaceutically acceptable salt thereof.

24. The agent of claim 23 wherein the antidiabetic drug is an insulin secretagogue.

25. The agent of claim 24 wherein the insulin secretagogue is a SU drug.

26. The agent of claim 24 wherein the insulin secretagogue is a phenylalanine derivative.

27. The agent of claim 24 wherein the insulin secretagogue is a GLP-1 analogue.

28. The agent of claim 24 wherein the insulin secretagogue is a DPP-4 inhibitor.

29. The agent of claim 23 wherein the antidiabetic drug is a non-insulin secretagogue.

30. The agent of claim 29 wherein the non-insulin secretagogue is a biguanide.

31. The agent of claim 29 wherein the non-insulin secretagogue is an α-glucosidase inhibitor.

32. A method for increasing the hypoglycemic effect of an antidiabetic drug in a mammal comprising administering to the mammal 2-methyl-2-[(4-{[(1E)-3-[2-(4-methylbenzoyl)-1H-pyrrol-1-yl]prop-1-en-1-yl}benzyl)oxy]propanoic acid or a pharmaceutically acceptable salt thereof.

33. Use of 2-methyl-2-[(4-{(1E)-3-[2-(4-methylbenzoyl)-1H-pyrrol-1-yl]prop-1-en-1-yl}benzyl)oxy]propanoic acid or a pharmaceutically acceptable salt thereof for the manufacture of an agent for increasing hypoglycemic effect of an antidiabetic drug.

34. A method for lowering blood glucose level in a mammal comprising administering to the mammal an antidiabetic drug and 2-methyl-2-[(4-{(1E)-3-[2-(4-methylbenzoyl)-1H-pyrrol-1-yl]prop-1-en-1-yl}benzyl)oxylpropanoic acid or a pharmaceutically acceptable salt thereof which lowers blood glucose level more effectively than when each of the said drugs is administered alone.
